# EUROPEAN PATENT APPLICATION

(11) **EP 2 784 165 A1**
(43) Date of publication of application: **01.10.2014**
(21) Application number: 13460020.4
(22) Date of filing: 29.03.2013
(51) Int. Cl.: C12Q 1/68

(54) **A method for detecting a genetic predisposition to prostate cancer (PC)**

(71) Applicant: Centrum Badan DNA Sp. z o.o., 61-612 Poznan (PL)
(72) Inventor: Wojciechowicz, Tatiana, 60-682 Poznan (PL); Bociag, Piotr, 62-082 Kozieglowy (PL); Odwazna, Joanna, 64-000 Koscian (PL); Wróblewska-Kabba, Sylwia, 60-688 Poznan (PL); Lacna, Katarzyna, 60-681 Poznan (PL); Sikorski, Adam, 89-652 Lag (PL); Wojciechowicz, Jacek, 60-682 Poznan (PL)
(74) Representative: Kossowska, Janina

(57) **Abstract**

The present invention relates to a method for detecting genetic predisposition to prostate cancer using DNA microarray by testing patient's biological sample towards occurance of mutations in genes RNASEL, ELAC2, MSR1, NBS1, BRCA1, CHEK2.

Further, the invention relates to a kit for carrying out the method according to the invention and a set of primers for use in the amplification of genetic material by PCR method and a set of oligonucleotide sequences for use as probes for detecting genetic predisposition to prostate cancer.

## Description

The present invention relates to a method for detecting genetic predisposition to prostate cancer, a kit for use in this method and a set of primers for PCR amplification of genomic DNA obtained from a patient as well as to oligonucleotide sequences for use as probes.

Prostate cancer (PC) is considered one of the most common tumors among men, constituting as much as 33% of newly diagnosed malignant tumors. PC incidence varies depending on the geographic region. The highest incidence is recorded in Canada, USA and Nordic countries, while the lowest is in China and Japan (Jemal A. et al. CA Cancer J Clin 61(2): 69-90, 2011).

According to reports of Maria Sklodowska-Curie Memorial Cancer Center and Institute in Warsaw, the risk of developing prostate cancer in men over a lifetime is 16.7%, but autopsy studies indicate that probability of occurance of histopathological features of PC is much higher. Studies have shown that prostate glands in 20% of men aged 50-60 and 50% of men aged 70-80 have subclinical changes with characteristics of malignancy. Most often they do not, however, cause a full-blown neoplastic disease (Carter B. et al. Nat Acad Sci 89: 3367-3371, 1992).

The above data put prostate cancer on the second place (after lung cancer) among causes of cancer deaths of men in Poland. Also the growth rate of PC morbidity and PC mortality is important. Since 1991, prostate cancer incidence has increased more than three-fold, and the accompanying mortality almost two-fold. The share of prostate cancer in general pool of cancer deaths has increased (Wojciechowska U., Didkowska J., Zatoński W., Centrum Onkologii Instytut Biuletyn 2008).

It is known that the prevalence of cancer may have a genetic basis, and therefore preventive examination should include testing an individual towards occurrence or lack of specific mutations in the genes (Cybulski C., et al. Postepy Nauk Medycznych 11: 846-850, 2011).

Genetic test result does not tell us about the current state of health. Determining the presence of a genetic mutation is not equivalent to the existence of the disease at a given time, it only shows an increased predisposition to the disease, a possibility of its development at a much earlier age, and what is related thereto, the need to implement appropriate prophylaxis. There is also a risk of transmitting a mutation to offspring. However, absence of a mutation is not equivalent to the fact that cancer is not developing or will never develop in a subject. Even people who have been excluded as carriers are vulnerable to adverse effects of environmental factors and the possibility of developing cancer in an older age.

Genetic test result is immutable, so there is no need to repeat the test. Due to the fact that the examined changes are the result of inheritance from a parent, it does not matter at which stage of life the test is carried out. The test result does not indicate a disease, it only indicates a possibility of its occurrence.

In connection with difficulties that may occur and in connection with often erroneous interpretation of a genetic test result it is indicated to visit a specialized genetic clinic with every genetic test result. Due to the fact that genetic tests are tests of a particular type and their results can have a huge impact on the personal life of patients and their families, only persons of legal age, after signing an informed consent for genetic studies, can undergo a test for genetic predispositions to tumors.

Familial prostate cancer has been observed for a long time, but it was only in 1992 that it was confirmed and described (Carter B. et al. Nat Acad Sci 89: 3367-3371, 1992). Earlier selection of carriers of specific changes in their genes, e.g. RNASEL, ELAC2, MSR1, NBS1, BRCA1 and CHEK2, where close relatives (I or II degree) had at least once case of prostate cancer, allows to monitor these persons and possibly to detect tumor earlier. It has been observed that specific changes in genes NBS1, BRCA1 and CHEK2 in the closest family increases the possibility of prostate cancer almost 5-15-times (Cybulski C. et al. Postepy Nauk Medycznych 11: 846-850, 2011).

Since prostate cancer initially develops without any clear symptoms, up to present basic diagnostic methods already included assaying PSA (*Prostate Specific Antigen*) in serum, palpation of prostate and transrectal ultrasonography. There are, however, prostate cancers, which do not cause an increase in the level of PSA in serum. Therefore there has been a need to develop another method for determining predisposition to prostate cancer (Heidenreich et al. European Urology 53 (1): 68-80, 2008).

Present invention meets this need and provides a new method for detecting predisposition to prostate cancer.

The method according to the present invention consists in identifying 17 key genetic mutations conditioning familial form of prostate cancer in a sample taken from a subject. This is the first comprehensive genetic test of this kind which detects the most of key mutations responsible for hereditary form of prostate cancer and is conducted using DNA microarray technology.

The DNA microarray technology is one of the most preferable methods developed in order to:
- analyse gene expression (detecting mRNA transcribed into more stable DNA);
- detect genetic material;
- analyse genomic DNA (so called genotyping).

A DNA microarray is a glass or plastic slide with appropriately prepared surface on which molecular probes were immobilized (usually these are chemically synthesized under laboratory conditions oligonucleotides complementary to a specific DNA fragment of the tested sample). The tested genetic material is attached to the probes specifically on the basis of complementarity (hybridization). Positions and fields that the probes occupy on a slide are defined in advance and they are in a matrix system (the fields form a regular arrangement of rows and columns). Each probe is specific for sequences of the analyzed genetic material for which it was designed in order to allow visualization (readout on a monitor connected to a microarray scanner) of signals with specific positions on the microarray, providing information on the analyzed changes in the analyzed genetic material. There are many types of microarrays offered by many manufacturers (including Affymetrix, Illumina, Roche, Perlan Bio, Genorama), which are related to differences in methodology, length of the probes, method of visualization of the signal, etc.

In the case of present invention the most preferable microarray platform turned out to be APEX (Arrayed Primer Extension) using microarray scanner Genorama QuattroImager (Berik J., Kurg A. Metspalu A., Estonian Bioct 2008: EP1980841). This platform is used only for genotyping - analysis of changes in genomic DNA (US Patent Application No. 09/741,960).

Analysis of genomic information from APEX microarray comprises the isolation of genetic material, amplification of selected regions of genomic DNA using designed primers, fragmentation of obtained amplification products and deactivation of unincorporated nucleotides, denaturation of obtained fragments to one strand form, hybridization of the fragmented amplification products with previously designed immobilized oligonucleotide probes and APEX reaction as well as visualization of the analyzed changes.

### Detailed description of the invention

The present invention relates to a method of carrying out a first complex genetic test to detect most of the key mutations responsible for hereditary prostate cancer. This test is intended for patients with cancer, their families and members of families in which prostate cancer occurs in close members of one family.

Inventors of present invention have combined results obtained in previous genetic studies of prostate cancer with a new DNA microarray technique, using a combination of primers and probes specific for selected locations in the human genome.

An advantage of this new method according to the invention is the ability to test more than 100 mutations simultaneously. This method requires a relatively short time to conduct and is very economical. It would also open the possibility of introducing new mutations to the test, in case new mutations or SNPs (*Single Nucleotide Polymorphism*) having an impact on occurrence of prostate cancer are disclosed.

The present invention relates to a method for detecting key mutations leading to prostate cancer and for determining predisposition to prostate cancer, comprising:
(a) obtaining a biological sample from a patient;
(b) isolating genomic DNA from a sample obtained from a patient;
(c) amplification of regions containing the analyzed mutation sites and polymorphic sites;
(d) purifying and concentrating the amplification products;
(e) fragmentation and functional inactivation of unincorporated nucleotides;
(f) hybridization of fragmented DNA obtained from a patient with oligonucleotide probes immobilized at specific points on the slide designed according to the nucleotide sequence of at least RNASEL, ELAC2, MSR1, NBS1, BRCA1, CHEK2 genes for sense and antisense directions (SE and AS) of sequence flanking the site of the genetic change;
(g) adding, on the principle of complementarity, a single ddNTP to the end of each probe on the DNA array;
(h) excitation of a slide with at least four lengths of light successively and reading signals by means of a DNA microarray scanner.

In present disclosure "obtaining a sample of genomic DNA from a patient" means taking a sample of his peripheral blood or swab of inner cheek surface.

In some embodiments obtained samples are stored short term at 4°C, in other embodiments samples are stored long term at -20°C or -70°C. Repeated freezing and thawing degrades DNA.

Methods of extracting gDNA from a sample are preferably carried out using genomic DNA isolation kits NucleoSpin Dx Blood and NucleoSpin Tissue (Marcherey-Nagel, Germany).

Obtained gDNA serves as a template for amplification of 14 regions of six analyzed genes RNASEL, ELAC2, MSR1, NBS1, BRCA1 and CHEK2, including 17 analyzed mutation sites and polymorphic sites.

The term "mutation" as used herein means, in accordance with its generally accepted meaning, any genetic change such as substitution, insertion or deletion, which can lead to malignancies.

The term "amplification" means multiplication in vitro of genetic material comprising selected regions of genomic DNA.

According to the present invention, amplification is preferably conducted by means of Multiplex PCR method using pairs of oligonucleotide primers and APEX Template Preparation Kit (Genorama, Estonia) to amplify sequence fragments of interest. It is essential in this step to design appropriate primers, without which the amplification step would be impossible.

Compared with conventional PCR part of dTTP fraction in PCR reaction mixture is replaced with 20% dUTP. Incorporation of dUTP allows subsequent fragmentation of the material with uracil-N-glycosylase (UNG), which allows efficient hybridization of short fragments of a product to oligonucleotide probes because obtained PCR products have a length from 100 to 1000 bp, preventing effective hybridization.

As used herein, "purification and concentration" means that products are pooled, concentrated and purified, preferably using GeneJet PCR kit (Genorama, Estonia), which consists of GeneJet columns for purification of PCR products, binding buffer, washing buffer and elution buffer.

Fragmentation and functional inactivation of unincorporated nucleotides is preferably conducted in a single incubation step at 37°C with the use of uracil-N-glycosylase (UNG) (Genorama, Estonia) and alkaline phosphatase (SAP or FastAP) (Genorama, Estonia) treatment. Uracil-N-glycosylase (UNG) recognizes uracil in dsDNA and ssDNA and hydrolyzes the bond between uracil and deoxyribose moiety. Samples are heated in order to inactivate enzymes and to conduct fragmentation of DNA in sites with uracil. Alkaline phosphatase inactivates nucleotides by removal of 5' phosphate moieties. Fragmentation of long PCR products is carried out in order to facilitate proper hybridization with oligonucleotides (probes) immobilized on a glass slide (APEX microarray).

Fragmentation is followed by denaturation of obtained fragments. Denaturation of DNA is separation of naturally occurring DNA double-strand at 95°C into a single strand form capable of hybridizing with complementary strand, including a nucleotide probe.

Oligonucleotides used as probes for microarrays are designed according to nucleotide sequence of RNASEL, ELAC2, MSR1, NBS1, BRCA1, CHEK2 genes for sense and antisense directions (SE and AS), including changes predisposing to prostate cancer:
- for RNASEL gene: p.E256X; p.M1I; p.R462Q and p.D541E;
- for ELAC2 gene: p.S217L; p.A541T; p.R781H;
- for MSR1 gene: p.P275A and p.R293X;.
- for NBS1 gene change c.657del5;
- for BRCA1 gene: c.185delAG; p.C61G; c.4153delA and c.5382insC;
- for CHEK2 gene: c.IVS2+1G>A; p.I157T and p.S428F.

Preferably, microarray used in the method according to the invention is APEX microarray.

Probes in APEX microarray are immobilized on a glass slide at their 5' terminal.

APEX oligonucleotides (probes) are designed so as to flank the tested genetic change.

As used herein, the term "APEX microarray" means a glass slide measuring 24 x 60 x 0.15 mm coated with aminosilane with a linker (SAL), on which fields with immobilized oligonucleotide probes are arranged in a matrix system at specific positions.

Microarray slides (Genorama, Estonia) stored at 4°C, are washed before use in distilled water at 95°C, to wash out buffer which is blocking the probes and to minimize background level before the APEX reaction.

Fragmented PCR products are denatured in a heating block at 95°C (10 min.) and then added to the APEX Reaction Mix (Genorama, Estonia) containing fluorescently labeled dideoxynucleotides (ddNTPs) (preferably FI-12-ddUTP, Cy3-ddCTP, Teras Red-ddATP, Cy5-ddGTP) and ThermoSequenase polymerase. Thus prepared mixture is applied on a microarray slide disposed in a heating block at a temperature of 58°C. Microarray is then covered with a glass coverslip LifterSlip (Genorama, Estonia) and incubated (58°C for 20 minutes). Hybrydyzation step of patient's fragmented DNA with microarray probes follows.

Hybridization consists in attaching fragmented amplification products with a probe immobilized on a slide in a complementary specific way.

Oligonucleotide probes specific for analyzed sites are designed so that each probe sequence ends just before the analyzed genetic change and its length is determined based on the melting point of a oligonucleotide single strand. Probes specific in terms of complementarity with analyzed DNA fragment hybridize at 58°C with fragmented amplification products, allowing APEX reaction. It occurs at the same temperature, and consists in extending of oligonucleotides (also called probes) arranged in line and immobilized on a glass slide by one fluorescently labeled nucleotide, using thermostable polymerase (an enzyme capable of binding nucleotides in accordance with the principle of complementarity in direction of 5'-> 3' after providing appropriate reaction conditions), while at the same time causing termination of extending of the oligonucleotide. Probes must be designed so that the covalently attached, fluorescently labeled dideoxynucleotide (terminal nucleotide) signals the presence or absence of mutations. Extension is carried out on the basis of complementarity with nucleotide in patient's DNA sequences, therefore specifically. Upon excitation of appropriately fluorescently labeled terminal nucleotides with appropriate wavelength of laser light, signal emitted by fluorochromes provides information on presence of specific nucleotide and thereby of a mutation or absence of a genetic change. For each mutation site the two types of probes are designed in most cases - sense probe (SE; designed on the basis of gDNA sense sequence) and antisense (AS; complementary to the sense strand, designed on the basis of gDNA antisense sequence). Probes are located on the microarray, each in duplicate. Because of that a slide comprises 4 fields of probes for each mutation sites - two fields with SE probes and two fields AS probes.

APEX reaction is terminated by placing the microarray slides in 0.3% Alcanox solution for 3 minutes. The slides are then washed and a drop of Atlas Antifade Reagent (Genorama, Estonia) is applied on the microarray, what prevents fading of fluorochromes.

Readout of signals is carried out using a microarray scanner, preferably Genorama Quattrolmager (Genorama, Estonia) equipped with 4 lasers with different wavelength ranges. Slides are excited in turns with four different lengths of laser light, causing excitation of fluorescently labeled nucleotides by the phenomenon of internal reflection.

Use of an CCD (Charge Coupled Device) image sensor allows registration of light signals from each of fluorochromes. Results are archived and analyzed using Genorama Genotyping Software (Genorama Estonia).

Subsequently verification of positive and doubtful signals is performed by DNA sequencing by means of chain termination method (in this case using BigDye Terminator Kit, Applied Biosystems, USA and ABI Prism 3100-Avant apparatus, Applied Biosystems, USA).

Using an appropriate compilation of specific, designed for the need of this diagnostic test, primers and oligonucleotide probes, enables innovative diagnostic of genetic predispositions using DNA microarray technology.

A list of indicated genes, loci thereof, a set of sequences with tested mutation sites and polymorphic sites in these genes and PCR primers designed for them are shown in Table 1.

**Table 1**

| **No** | **GENE/LOCUS** | **Analyzed change** | **Position on microarray** | **Designation of primers and mutations** | | **Name of a gene sequence record from ENSEMBL database** |
|---|---|---|---|---|---|---|
| | | | | **SE** | **AS** | |
| 1 | RNASEL/1q25.3 (OMIM) | p.E256X | 1.3 | PCR-RNASEL_2 RNASEL_E265X_SE GT (where G is a correct signal and T is a mutation signal | PCR-RNASEL_2 RNASEL_E265X_AS CA | ENST00000367559 |
| 2 | RNASEL/1q25.3 (OMIM) | P.M1I | 1.5 | PCR-RNASEL_1 RNASEL_M1I_SE GA | PCR-RNASEL_1 RNASEL_M1I_AS CT | ENST00000367559 |
| 3 | RNASEL/1q25.3 (OMIM) | p.R462Q | 1.7 | PCR-RNASEL_2 RNASEL_R462Q_SE GA | PCR-RNASEL_2 RNASEL_R462Q_AS CT | ENST00000367559 |
| 4 | RNASEL/1q25.3 (OMIM) | p.D541E | 1.9 | PCR-RNASEL_3 RNASEL_D541E_SE TG | PCR-RNASEL_3 RNASEL_D541E_AS AC | ENST00000367559 |
| 5 | ELAC2/17p12 (OMIM) | p.S217L | 3.1 | PCR-ELAC2_1 ELAC2_S217L_SE CT | PCR-ELAC2_1 ELAC2_S217L_AS GA | ENST00000338034 |
| 6 | ELAC2/17p12 (OMIM) | p.A541T | 3.3 | PCR-ELAC2_2 ELAC2_A541T_SE GA | PCR-ELAC2_2 ELAC2-A541T_AS CT | ENST00000338034 |
| 7 | ELAC2/17p12 (OMIM) | p.R781H | 3.5 | PCR-ELAC2_1 ELAC2_R781H_SE GA | PCR-ELAC2_3 ELAC2_R781H_AS CT | ENST00000338034 |
| 8 | MSR1/8p22 (OMIM) | p.P275A | 3.7 | PCR- MSR1_1 MSR1_P275A_SE CG | PCR- MSR1_1 MSR1_P275A_AS GC | ENST00000262101 |
| 9 | MSR1/8p22 (OMIM) | P.R293X | 3.9 | PCR- MSR1_1 MSR1_R293X_SE GA | PCR-MSR1_1 MSR1_R293X_AS CT | ENST00000262101 |
| 10 | NBS1/8q21 (OMIM) | c.657del5 | 3.11 | PCR-NSB1_1 NBS1_657del5_SE AT | PCR-NSB1_1 NBS1_657del5_AS GC | ENSG00000104320 |
| 11 | BRCA1/17q21 (OMIM) | c.185delAG | 5.1 | PCR- BRCA1_1 BRCA1_185delAG_SE AT | PCR-BRCA1_1 BRCA1-185delAG_AS CA | ENST00000357654 |
| 12 | BRCA1/17q21 (OMIM) | p.C61G | 5.3 | PCR- BRCA1_2 BRCA1_C61G_SE TG | PCR-BRCA1_2 BRCA1_C61G_AS AC | ENST00000357654 |
| 13 | BRCA1/17q21 (OMIM) | c.4153delA | 5.5 | PCR- BRCA1_3 BRCA1_4153delA_SE AG | PCR- BRCA1_3 BRCA1_4153delA_AS TC | ENST00000357654 |
| 14 | BRCA1/17q21 (OMIM) | c.5382insC | 5.7 | PCR- BRCA1_4 BRCA1_5382insC_SE CA | PCR- BRCA1_4 BRCA1_5382insC_AS GA | ENST00000357654 |
| 15 | CHEK2/22q12.1 (OMIM) | c.IVS2+1G>A | 5.9 | PCR- CHEK2_1 CHEK2_IVS2+1G>A_SE GA | PCR-CHEK2_1 CHEK2_IVS2+1G>A_AS CT | ENST00000328354 |
| 16 | CHEK2/22q12.1 (OMIM) | p.I157T | 5.11 | PCR- CHEK2_2 CHEK2_I157T_SE TC | PCR-CHEK2_2 CHEK2_I157T_AS AG | ENST00000328354 |
| 17 | CHEK2/22q12.1 (OMIM) | p.S428F | 7.3 | PCR- CHEK2_3 CHEK2_S428F_SE CT | PCR- CHEK2_3 CHEK2_S428F_AS GA | ENST00000328354 |

The present invention also relates to a kit for carrying out the method according to the invention which comprises:
a) DNA microarrays with immobilized oligonucleotides - type SE and AS probes presented in SEQ ID NOS: 1-34;
b) PCR primers shown in SEQ ID NOS: 35-68 for carrying out amplification of DNA template fragments comprising tested changes;
c) PCR reagents;
d) APEX mixture;
e) slip covers;
f) washing reagent for slides;
g) protocol.

PCR reagents preferably comprise polymerase, reaction buffor, dNTPs (including 20% UTP), MgCl₂.

APEX mixture preferably comprises ThermoSequenase polymerase, fluorescently labeled dideoxynucleotides: FL-12-ddUTP; Cy3-ddCTP, Texas Red-ddATP, Cy5-ddGTP.

The kit of the present invention includes primers designed for amplification of sequence fragments derived from the patient's DNA template.

DNA template fragments of interest are fragments comprising regions with analyzed mutation sites or polymorphic sites in the above mentioned genes. Analyzed sites have been selected on the basis of available literature. So far one particular gene, which is responsible for triggering prostate cancer has not been known. Recent reports on correlation of genetic changes and prostate cancer are updated in the OMIM database (*www.ncbi.nlm.nih.gov*/*omim*).

In studies devoted to the search for genes associated with occurrence of prostate cancer, much attention is paid to changes occurring in CHEK2 gene. This gene encodes CHECK2 protein which is effector kinase involved in DNA repair. Literature research reports three polymorphic variants associated with increased risk of prostate cancer in the Polish population (Cybulski et al. Cancer Research 64: 121-52, 2004).

Another gene which plays a critical role in DNA repair, cell cycle control and maintaining genomic stability is BRCA1 gene. (Giusti et al. J. Med. Genet. 40: 787-792, 2003; Górski B. et al. American Journal of Human Genetics 66 (6): 1963-1968, 2000; Perkowska M et al. Hum Mutat 21(5): 553-4, 2003).

The relationship between ELAC2 gene and prostate cancer was reported in Rebbeck TR et al. (Rebbeck TR et al. Am J Hum Genet 67 (4): 1014-9, 2000).

The next gene considered in case of prostate cancer is RNASEL gene encoding 2-5A-dependent RNase (ribonuclease 4), which is a component of 2-5A interferon controlling system and affecting antiviral and antiproliferative interferon function (Rokman, A et al. Am. J . Hum. Genet. 70: 1299-1304, 2002; Carpten J. et al. Nature Genet 30: 181-184, 2002; Casey G et al. Nature. Genet 32: 581-583, 2002).

Another gene which is relevant in determining predisposition to prostate cancer, is MSR1 gene encoding a protein of macrophage scavenger receptors. These receptors are involved in binding, internalization and processing of negatively charged macromolecules (Xu J et al. Nature Genet 32: 321-325, 2002).

Yet another gene considered in case of prostate cancer is NBS1 gene. The protein product encoded by this gene is fibrin involved in reparation of damage in dsDNA. Studies have shown that mutations in NBS1 gene are the cause of about 3% of all prostate cancers and about 9% of familial prostate cancer cases (Cybulski et al. Cancer Research 64: 121-52, 2004).

Present invention relates also to a primer set for use in PCR amplification of genetic material. Primers according to the invention include nucleotide sequences presented as SEQ ID NOS: 35-68.

The scope of present invention also includes oligonucleotide sequences presented as SEQ ID NOS: 1-34 for use as probes for detecting genetic predisposition to prostate cancer. The probes are designed to include SE and AS probes and may be used in other methods for hybridization of tested material.

The following example is provided for better understanding of the present invention, the scope of which is set forth in the appended claims.

### Example

The tests were performed on a group of 8 terminal patients with prostate cancer. All patients underwent an innovative test of genetic predisposition for development of prostate cancer based on APEX microarray technology. Analyzed patients constituted a validation group of genetic predisposition test for prostate cancer. Peripheral blood sample was taken from each patient and subjected to genetic test.

Individual stages of the analysis are shown in an example of one patient from the validation group.

By means of the filed innovative method of diagnosis of genetic predispositions, including analysis of selected 17 sites in the human genome associated with genetic predisposition to prostate cancer, revealed the presence of four genetic variations in the genes: RNASEL (c.1385G> A in a heterozygous configuration c.1623T> G in heterozygous configuration) ELAC2 (c.650C> T in heterozygous configuration) and MSR1 (c.823C> G in homozygous configuration).

### Preparation of material

Material collected for this study was patient's peripheral blood. Patient's genomic DNA was isolated from 200 µl of peripheral blood supplemented with EDTA. Isolation of gDNA was conducted using NucleoSpin Blood Dx Kit (Marcherey-Nagel, Germany). The obtained genetic material, suspended in 100 ml of elution buffer, was used as a template to investigate 14 regions of 6 analyzed genes (RNASEL, ELAC2, MSR1, NBS1, BRCA1, CHEK2), including 17 analyzed mutation sites and polymorphic sites.

### Amplification of material by PCR method

Amplification was conducted by means of Multiplex PCR method using 14 primer pairs and APEX Template Preparation amplification kit (Genorama, Estonia). In comparison with conventional PCR reaction, 20% fraction of dTTP in reaction mixture was replaced with dUTP. Amplification products were purified and concentrated using GeneJet PCR Kit (Genorama, Estonia).

### Fragmentation of the PCR product

Fragmentation and functional inactivation of unincorporated nucleotides were conducted in a single step using uracil N-glycosylase (Genorama, Estonia) and shrimp alkaline phosphatase (Genorama, Estonia).

### APEX reaction

In accordance with the description concerning the filed diagnostic test of genetic predisposition based on APEX microarray, oligonucleotides used as specific probes of the microarray, designed according to nucleotide sequences of genes RNASEL, ELAC2, MSR1, NBS1, BRCA1, CHEK2 for sense and antisense directions (SE and AS), enabled analysis of changes in genetic material of a patient. There were two types of probes: SE and AS for one analyzed mutation site, each in duplicate. Microarray slides (Genorama, Estonia) stored at 4°C, were labeled and washed in distilled water at 95°C before use to remove buffer blocking the probes and to minimize background level before the APEX reaction (*Arrayed Primer Extension*). PCR products fragmented in an earlier step were denatured in a heating block at 95°C (10 min.) and then added to the APEX reaction mixture (Genorama, Estonia) containing fluorescently labeled ddNTPs (Fl-12-ddUTP, Cy3-ddCTP, Texas Red-ddATP, Cy5-ddGTP) and ThermoSequenase polymerase. Thus prepared mixture was applied to a microarray slide and placed in a heating block at a temperature of 58°C. The microarray was covered with LifterSlip coverslip (Genorama, Estonia) and incubated (58°C, 20 min.). The reaction was terminated by placing the APEX microarray slides in 0.3% Alcanox solution (3 min.), then the plates were washed three times with MilliQ water (95°C, 1 min.). A droplet of Atlas Antifade Reagent (Genorama, Estonia) were applied on the microarray to prevent fading of fluorochromes.

### Readout of results

Signals readout was conducted using a microarray scanner Genorama QuattroImager (Genorama, Estonia). Thanks to usage of CCD (Charge Coupled Device) image sensor it was possible to record light signals of individual fluorochromes. The results archived in TIFF format and analyzed using Genorama Genotyping Software (Genorama, Estonia).

Positive signals from the microarray were verified by DNA sequencing by method of chain termination, using BigDye Terminator v.3.1 Cycle Sequencing Kit (Applied Biosystems, USA) and ABI Prism 3100-Avant (Applied Biosystems, USA) apparatus to confirm changes detected by an alternative test method.

## Claims

1. A method for detecting genetic predisposition to prostate cancer using DNA microarray, comprising:
(a) obtaining a biological sample from a patient;
(b) isolating genomic DNA from the sample obtained from a patient;
(c) amplification of regions containing analyzed mutation sites and polymorphic sites;
(d) purification and concentration of amplification products;
(e) fragmentation and functional inactivation of unincorporated nucleotides;
(f) hybridization of fragmented DNA obtained from a patient with oligonucleotide probes immobilized at specific points on a slide, designed in accordance with nucleotide sequences of at least RNASEL ELAC2, MSR1, NBS1, BRCA1, CHEK2 genes for sense and antisense directions (SE and AS) of a sequence flanking genetic change site;
(g) adding, on the principle of complementarity, a single ddNTP to the end of each probe on the DNA microarray;
(h) excitation of a slide successively with at least four lengths of light and reading signals by means of a microarray scanner.

2. Method according to claim 1, wherein the sample taken from a patient is a blood sample.

3. Method according to claim 1, wherein the sample taken from a patient is a swab from inner cheek surface.

4. Method according to claim 1, wherein amplification is conducted by means of PCR method using primers shown in SEQ ID NOS: 35-68.

5. Method according to claim 1, wherein probes having sequences shown in SEQ ID NOS: 1-34 are used.

6. Method according to claim 1, wherein the microarray is APEX microarray and microarray analysis is conducted using an APEX scanner.

7. Method according to claim 1, wherein mutations in RNASEL gene are at least mutations: p.E256X; p.M1I; p.R462Q and p.D541E.

8. Method according to claim 1, wherein mutations in ELAC2 gene are at least mutations: p.S217L; p.A541T; p.R781H.

9. Method according to claim 1, wherein mutations in MSR1 gene are at least mutations: p.P275A and p.R293X.

10. Method according to claim 1, wherein the mutation in NBS1 is at least mutation c.657del5.

11. Method according to claim 1, wherein mutations in BRCA1 gene are at least mutations: c.185delAG; p.C61G; c.4153delA and c.5382insC.

12. Method according to claim 1, wherein mutations in wherein mutations in CHEK2 gene are at least mutations: c.IVS2+1G>A; p.I157T and p.S428F.

13. Kit for carrying out the method according to claim 1, which comprises:
a) DNA microarrays with immobilized oligonucleotides - type SE and AS probes presented in SEQ ID NOS: 1-34:
b) PCR primers shown in SEQ ID NOS: 35-68 for carrying out amplification of DNA matrix fragments comprising tested changes;
c) PCR reagents;
d) APEX mixture;
e) slip covers;
f) washing reagent for slides;
g) protocol.

14. A set of primers shown in SEQ ID NO: 35-68 for use in the amplification of the genetic material by means of PCR method.

15. Oligonucleotide sequences shown as SEQ NO: 1-34 for use as probes for detecting genetic predisposition to prostate cancer.


SEQUENCE LISTING

<110> Centrum Badan DNA Sp. z o. o.

<120> A method for detecting a genetic predisposition to prostate cancer
(PC)

<160> 68

<170> BiSSAP 1.2

<210> 1
<211> 25
<212> DNA
<213> Artificial Sequence

<220>
<221> source
<222> 1..25
<223> /mol_type="unassigned DNA"
      /note="Oligonucleotide sense probe designed on
      the basis of RNASEL gene sequence for p.E256X site"
      /organism="Artificial Sequence"

<400> 1
gaggcttctg gagcaagagc acata                                          25


<210> 2
<211> 28
<212> DNA
<213> Artificial Sequence

<220>
<221> source
<222> 1..28
<223> /mol_type="unassigned DNA"
      /note="Oligonucleotide antisense probe designed on
      the basis of RNASEL gene sequence for p.E256X site"
      /organism="Artificial Sequence"

<400> 2
ttgccatcac tgtctgtgtc attaatct                                       28


<210> 3
<211> 25
<212> DNA
<213> Artificial Sequence

<220>
<221> source
<222> 1..25
<223> /mol_type="unassigned DNA"
      /note="Oligonucleotide sense probe designed on
      the basis of RNASEL gene sequence for p.M1I site"
      /organism="Artificial Sequence"

<400> 3
ggtagcaggt ggcatttacc gtcat                                          25


<210> 4
<211> 24
<212> DNA
<213> Artificial Sequence

<220>
<221> source
<222> 1..24
<223> /mol_type="unassigned DNA"
      /note="Oligonucleotide antisense probe designed on
      the basis of RNASEL gene sequence for p.M1I site"
      /organism="Artificial Sequence"

<400> 4
ggggttgtta tgatccctgc tctc                                           24


<210> 5
<211> 26
<212> DNA
<213> Artificial Sequence

<220>
<221> source
<222> 1..26
<223> /mol_type="unassigned DNA"
      /note="Oligonucleotide sense probe designed on
      the basis of RNASEL gene sequence for p.R462Q site"
      /organism="Artificial Sequence"

<400> 5
ggaaaatgag gaagatgaat ttgccc                                         26


<210> 6
<211> 32
<212> DNA
<213> Artificial Sequence

<220>
<221> source
<222> 1..32
<223> /mol_type="unassigned DNA"
      /note="Oligonucleotide antisense probe designed on
      the basis of RNASEL gene sequence for p.R462Q site"
      /organism="Artificial Sequence"

<400> 6
gaacagcctt aaatatagat gacaggacat tt                                  32


<210> 7
<211> 29
<212> DNA

<213> Artificial Sequence

<220>
<221> source
<222> 1..29
<223> /mol_type="unassigned DNA"
      /note="Oligonucleotide sense probe designed on
      the basis of RNASEL gene sequence for p.D541E site"
      /organism="Artificial Sequence"

<400> 7
gtaaagaagg gaagcatctc atttgagga                                         29


<210> 8
<211> 30
<212> DNA
<213> Artificial Sequence

<220>
<221> source
<222> 1..30
<223> /mol_type="unassigned DNA"
      /note="Oligonucleotide antisense probe designed on
      the basis of RNASEL gene sequence for p.D541E site"
      /organism="Artificial Sequence"

<400> 8
aaccacctct tcattacttt gagctttcag                                        30


<210> 9
<211> 25
<212> DNA
<213> Artificial Sequence

<220>
<221> source
<222> 1..25
<223> /mol_type="unassigned DNA"
      /note="Oligonucleotide sense probe designed on
      the basis of ELAC2 gene sequence for p.S217L site"
      /organism="Artificial Sequence"

<400> 9
ccagagcgat cttcagaatc cgagt                                             25


<210> 10
<211> 26
<212> DNA
<213> Artificial Sequence

<220>
<221> source
<222> 1..26
<223> /mol_type="unassigned DNA"
      /note="Oligonucleotide antisense probe designed on

the basis of ELAC2 gene sequence for p.S217L site"
      /organism="Artificial Sequence"

<400> 10
gtggaaggtg tggctcattt tcattc                                          26


<210> 11
<211> 18
<212> DNA
<213> Artificial Sequence

<220>
<221> source
<222> 1..18
<223> /mol_type="unassigned DNA"
      /note="Oligonucleotide sense probe designed on
      the basis of ELAC2 gene sequence for p.A541T site"
      /organism="Artificial Sequence"

<400> 11
gtcctgggca ccctggct                                                   18


<210> 12
<211> 22
<212> DNA
<213> Artificial Sequence

<220>
<221> source
<222> 1..22
<223> /mol_type="unassigned DNA"
      /note="Oligonucleotide antisense probe designed on
      the basis of ELAC2 gene sequence for p.A541T site"
      /organism="Artificial Sequence"

<400> 12
tgcaggtggg acacaaacac ag                                              22


<210> 13
<211> 22
<212> DNA
<213> Artificial Sequence

<220>
<221> source
<222> 1..22
<223> /mol_type="unassigned DNA"
      /note="Oligonucleotide sense probe designed on
      the basis of ELAC2 gene sequence for p.R781H site"
      /organism="Artificial Sequence"

<400> 13
gacatcgagg agatggagga gc                                              22

<210> 14
<211> 22
<212> DNA
<213> Artificial Sequence

<220>
<221> source
<222> 1..22
<223> /mol_type="unassigned DNA"
      /note="Oligonucleotide antisense probe designed on
      the basis of ELAC2 gene sequence for p.R781H site"
      /organism="Artificial Sequence"

<400> 14
ccgcagctcc cgcttctccc tg                                              22


<210> 15
<211> 38
<212> DNA
<213> Artificial Sequence

<220>
<221> source
<222> 1..38
<223> /mol_type="unassigned DNA"
      /note="Oligonucleotide sense probe designed on
      the basis of MSR1 gene sequence for p.P275A site"
      /organism="Artificial Sequence"

<400> 15
caactatgat cagtaactgt aattttttta aaggtcct                             38


<210> 16
<211> 22
<212> DNA
<213> Artificial Sequence

<220>
<221> source
<222> 1..22
<223> /mol_type="unassigned DNA"
      /note="Oligonucleotide antisense probe designed on
      the basis of MSR1 gene sequence for p.P275A site"
      /organism="Artificial Sequence"

<400> 16
ccttttcac ccgggggtcc ag                                               22


<210> 17
<211> 24
<212> DNA
<213> Artificial Sequence

<220>
<221> source

<222> 1..24
<223> /mol_type="unassigned DNA"
      /note="Oligonucleotide sense probe designed on
      the basis of MSR1 gene sequence for p.R293X site"
      /organism="Artificial Sequence"

<400> 17
gtcccactgg agaaaatggt ccac                                              24


<210> 18
<211> 28
<212> DNA
<213> Artificial Sequence

<220>
<221> source
<222> 1..28
<223> /mol_type="unassigned DNA"
      /note="Oligonucleotide antisense probe designed on
      the basis of MSR1 gene sequence for p.R293X site"
      /organism="Artificial Sequence"

<400> 18
taccatacct attggacctg gaaatcct                                          28


<210> 19
<211> 25
<212> DNA
<213> Artificial Sequence

<220>
<221> source
<222> 1..25
<223> /mol_type="unassigned DNA"
      /note="Oligonucleotide sense probe designed on
      the basis of NBS1 gene sequence for p.657del5 site"
      /organism="Artificial Sequence"

<400> 19
atctgtcagg acggcaggaa agaaa                                             25


<210> 20
<211> 33
<212> DNA
<213> Artificial Sequence

<220>
<221> source
<222> 1..33
<223> /mol_type="unassigned DNA"
      /note="Oligonucleotide antisense probe designed on
      the basis of NBS1 gene sequence for c.657del5 site"
      /organism="Artificial Sequence"

<400> 20

tcaaaaatat aaatgttttc cctttgaaga ttt                                    33

<210> 21
<211> 34
<212> DNA
<213> Artificial Sequence

<220>
<221> source
<222> 1..34
<223> /mol_type="unassigned DNA"
      /note="Oligonucleotide sense probe designed on
      the basis of BRCA1 gene sequence for c.185delAG site"
      /organism="Artificial Sequence"

<400> 21
caaaatgtca ttaatgctat gcagaaaatc ttag                                  34

<210> 22
<211> 28
<212> DNA
<213> Artificial Sequence

<220>
<221> source
<222> 1..28
<223> /mol_type="unassigned DNA"
      /note="Oligonucleotide antisense probe designed on
      the basis of BRCA1 gene sequence for c.185delAG site"
      /organism="Artificial Sequence"

<400> 22
cttgtgctga cttaccagat gggacact                                         28

<210> 23
<211> 25
<212> DNA
<213> Artificial Sequence

<220>
<221> source
<222> 1..25
<223> /mol_type="unassigned DNA"
      /note="Oligonucleotide sense probe designed on
      the basis of BRCA1 gene sequence for c.C61G site"
      /organism="Artificial Sequence"

<400> 23
caaccagaag aaagggcctt cacag                                            25

<210> 24
<211> 42
<212> DNA
<213> Artificial Sequence

<220>
<221> source
<222> 1..42
<223> /mol_type="unassigned DNA"
      /note="Oligonucleotide antisense probe designed on
      the basis of BRCA1 gene sequence for c.C61G site"
      /organism="Artificial Sequence"

<400> 24
aaattatata ccttttggtt atatcattct tacataaagg ac                    42


<210> 25
<211> 29
<212> DNA
<213> Artificial Sequence

<220>
<221> source
<222> 1..29
<223> /mol_type="unassigned DNA"
      /note="Oligonucleotide sense probe designed on
      the basis of BRCA1 gene sequence for c.4153delA site"
      /organism="Artificial Sequence"

<400> 25
agtgacaagg aattggtttc agatgatga                                     29


<210> 26
<211> 25
<212> DNA
<213> Artificial Sequence

<220>
<221> source
<222> 1..25
<223> /mol_type="unassigned DNA"
      /note="Oligonucleotide antisense probe designed on
      the basis of BRCA1 gene sequence for c.4153delA site"
      /organism="Artificial Sequence"

<400> 26
ttcttccaag cccgttcctc tttct                                         25


<210> 27
<211> 24
<212> DNA
<213> Artificial Sequence

<220>
<221> source
<222> 1..24
<223> /mol_type="unassigned DNA"
      /note="Oligonucleotide sense probe designed on
      the basis of BRCA1 gene sequence for c.5382insC site"

/organism="Artificial Sequence"

<400> 27
ggtccaaagc gagcaagaga atcc                                              24


<210> 28
<211> 26
<212> DNA
<213> Artificial Sequence

<220>
<221> source
<222> 1..26
<223> /mol_type="unassigned DNA"
      /note="Oligonucleotide antisense probe designed on
      the basis of BRCA1 gene sequence for c.5382insC site"
      /organism="Artificial Sequence"

<400> 28
ggagggagct ttactttctg tcctgg                                            26


<210> 29
<211> 27
<212> DNA
<213> Artificial Sequence

<220>
<221> source
<222> 1..27
<223> /mol_type="unassigned DNA"
      /note="Oligonucleotide sense probe designed on
      the basis of CHEK2 gene sequence for c.IVS2+1G>A site"
      /organism="Artificial Sequence"

<400> 29
agcaagaaac actttcggat tttcagg                                           27


<210> 30
<211> 34
<212> DNA
<213> Artificial Sequence

<220>
<221> source
<222> 1..34
<223> /mol_type="unassigned DNA"
      /note="Oligonucleotide antisense probe designed on
      the basis of CHEK2 gene sequence for c.IVS2+1G>A site"
      /organism="Artificial Sequence"

<400> 30
cagctctcct agatacatgg gtattcatta ccta                                   34


<210> 31

<211> 36
<212> DNA
<213> Artificial Sequence

<220>
<221> source
<222> 1..36
<223> /mol_type="unassigned DNA"
      /note="Oligonucleotide sense probe designed on
      the basis of CHEK2 gene sequence for p.I157T site"
      /organism="Artificial Sequence"

<400> 31
ctctatttta agaagtgggt cctaaaaact cttaca                                36


<210> 32
<211> 29
<212> DNA
<213> Artificial Sequence

<220>
<221> source
<222> 1..29
<223> /mol_type="unassigned DNA"
      /note="Oligonucleotide antisense probe designed on
      the basis of CHEK2 gene sequence for p.I157T site"
      /organism="Artificial Sequence"

<400> 32
cattgccact gtgatcttct atgtatgca                                       29


<210> 33
<211> 32
<212> DNA
<213> Artificial Sequence

<220>
<221> source
<222> 1..32
<223> /mol_type="unassigned DNA"
      /note="Oligonucleotide sense probe designed on
      the basis of CHEK2 gene sequence for p.S428F site"
      /organism="Artificial Sequence"

<400> 33
aatattaagc cttagtgggt atccaccttt ct                                    32


<210> 34
<211> 27
<212> DNA
<213> Artificial Sequence

<220>
<221> source
<222> 1..27

<223> /mol_type="unassigned DNA"
      /note="Oligonucleotide antisense probe designed on
      the basis of CHEK2 gene sequence for p.S428F site"
      /organism="Artificial Sequence"

<400> 34
ttcagtgaca cttgagtcct atgctca                                          27


<210> 35
<211> 20
<212> DNA
<213> Artificial Sequence

<220>
<221> source
<222> 1..20
<223> /mol_type="unassigned DNA"
      /note="Oligonucleotide forward primer designed on
      the basis of RNASEL gene sequence enabling amplification of
      a region comprising genetic change p.E256X site"
      /organism="Artificial Sequence"

<400> 35
catggggctg atgtcaatgt                                                  20


<210> 36
<211> 20
<212> DNA
<213> Artificial Sequence

<220>
<221> source
<222> 1..20
<223> /mol_type="unassigned DNA"
      /note="Oligonucleotide reverse primer designed
      on the basis of RNASEL gene sequence enabling amplification of
      a region comprising genetic change p.E256X site"
      /organism="Artificial Sequence"

<400> 36
tggtgggtgt atccacagga                                                  20


<210> 37
<211> 20
<212> DNA
<213> Artificial Sequence

<220>
<221> source
<222> 1..20
<223> /mol_type="unassigned DNA"
      /note="Oligonucleotide forward primer designed on
      the basis of RNASEL gene sequence enabling amplification of
      a region comprising genetic change p.M1I site"
      /organism="Artificial Sequence"

<400> 37
gttgccaggt ggaatgtcag                                                   20


<210> 38
<211> 20
<212> DNA
<213> Artificial Sequence

<220>
<221> source
<222> 1..20
<223> /mol_type="unassigned DNA"
      /note="Oligonucleotide reverse primer designed
      on the basis of RNASEL gene sequence enabling amplification of
      a region comprising genetic change p.M1I site"
      /organism="Artificial Sequence"

<400> 38
ccgctcttga tcctcctttg                                                   20


<210> 39
<211> 20
<212> DNA
<213> Artificial Sequence

<220>
<221> source
<222> 1..20
<223> /mol_type="unassigned DNA"
      /note="Oligonucleotide forward primer designed on
      the basis of RNASEL gene sequence enabling amplification of
      a region comprising genetic change p.R462Q site"
      /organism="Artificial Sequence"

<400> 39
catggggctg atgtcaatgt                                                   20


<210> 40
<211> 20
<212> DNA
<213> Artificial Sequence

<220>
<221> source
<222> 1..20
<223> /mol_type="unassigned DNA"
      /note="Oligonucleotide reverse primer designed
      on the basis of RNASEL gene sequence enabling amplification of
      a region comprising genetic change p.R462Q site"
      /organism="Artificial Sequence"

<400> 40
tggtgggtgt atccacagga                                                   20

<210> 41
<211> 22
<212> DNA
<213> Artificial Sequence

<220>
<221> source
<222> 1..22
<223> /mol_type="unassigned DNA"
      /note="Oligonucleotide forward primer designed on
      the basis of RNASEL gene sequence enabling amplification of
      a region comprising genetic change p.D541E site"
      /organism="Artificial Sequence"

<400> 41
ggcttgattt atggcttttg tg                                              22


<210> 42
<211> 21
<212> DNA
<213> Artificial Sequence

<220>
<221> source
<222> 1..21
<223> /mol_type="unassigned DNA"
      /note="Oligonucleotide reverse primer designed
      on the basis of RNASEL gene sequence enabling amplification of
      a region comprising genetic change p.D541E site"
      /organism="Artificial Sequence"

<400> 42
ctgtggcatc tgcacaaagt t                                               21


<210> 43
<211> 21
<212> DNA
<213> Artificial Sequence

<220>
<221> source
<222> 1..21
<223> /mol_type="unassigned DNA"
      /note="Oligonucleotide forward primer designed on
      the basis of ELAC2 gene sequence enabling amplification of a region
      comprising a mutation change p.S217L site"
      /organism="Artificial Sequence"

<400> 43
tcagctcacc ttgtgcagtg t                                               21


<210> 44
<211> 21
<212> DNA

<213> Artificial Sequence

<220>
<221> source
<222> 1..21
<223> /mol_type="unassigned DNA"
      /note="Oligonucleotide reverse primer designed
      on the basis of ELAC2 gene sequence enabling amplification of
      a region comprising genetic change p.S217L site"
      /organism="Artificial Sequence"

<400> 44
caagcctttc tgctgctctg t                                                21


<210> 45
<211> 21
<212> DNA
<213> Artificial Sequence

<220>
<221> source
<222> 1..21
<223> /mol_type="unassigned DNA"
      /note="Oligonucleotide forward primer designed on
      the basis of ELAC2 gene sequence enabling amplification of a region
      comprising a mutation change p.A541T site"
      /organism="Artificial Sequence"

<400> 45
atcagcctct gaaccatcag c                                                21


<210> 46
<211> 21
<212> DNA
<213> Artificial Sequence

<220>
<221> source
<222> 1..21
<223> /mol_type="unassigned DNA"
      /note="Oligonucleotide reverse primer designed
      on the basis of ELAC2 gene sequence enabling amplification of
      a region comprising genetic change p.A541T site"
      /organism="Artificial Sequence"

<400> 46
cctctctctt gcaggagttg c                                                21


<210> 47
<211> 20
<212> DNA
<213> Artificial Sequence

<220>
<221> source

<222> 1..20
<223> /mol_type="unassigned DNA"
      /note="Oligonucleotide forward primer designed on
      the basis of ELAC2 gene sequence enabling amplification of a region
      comprising a mutation change p.R781H site"
      /organism="Artificial Sequence"

<400> 47
tcatctgctt gcttccgtgt                                                   20


<210> 48
<211> 20
<212> DNA
<213> Artificial Sequence

<220>
<221> source
<222> 1..20
<223> /mol_type="unassigned DNA"
      /note="Oligonucleotide reverse primer designed
      on the basis of ELAC2 gene sequence enabling amplification of
      a region comprising genetic change p.R781H site"
      /organism="Artificial Sequence"

<400> 48
tcatctgctt gcttccgtgt                                                   20


<210> 49
<211> 24
<212> DNA
<213> Artificial Sequence

<220>
<221> source
<222> 1..24
<223> /mol_type="unassigned DNA"
      /note="Oligonucleotide forward primer designed on
      the basis of MSR1 gene sequence enabling amplification of a region
      comprising a mutation change p.P275A site"
      /organism="Artificial Sequence"

<400> 49
ggttgtttac tttgaggcaa tgtc                                              24


<210> 50
<211> 23
<212> DNA
<213> Artificial Sequence

<220>
<221> source
<222> 1..23
<223> /mol_type="unassigned DNA"
      /note="Oligonucleotide reverse primer designed
      on the basis of MSR1 gene sequence enabling amplification of

a region comprising genetic change p.P275A site"
      /organism="Artificial Sequence"

<400> 50
caatcctccc ctacacatgt acc                                              23


<210> 51
<211> 24
<212> DNA
<213> Artificial Sequence

<220>
<221> source
<222> 1..24
<223> /mol_type="unassigned DNA"
      /note="Oligonucleotide forward primer designed on
      the basis of MSR1 gene sequence enabling amplification of a region
      comprising a mutation change p.R293X site"
      /organism="Artificial Sequence"

<400> 51
ggttgtttac tttgaggcaa tgtc                                             24


<210> 52
<211> 23
<212> DNA
<213> Artificial Sequence

<220>
<221> source
<222> 1..23
<223> /mol_type="unassigned DNA"
      /note="Oligonucleotide reverse primer designed
      on the basis of MSR1 gene sequence enabling amplification of
      a region comprising genetic change p.R293X site"
      /organism="Artificial Sequence"

<400> 52
caatcctccc ctacacatgt acc                                              23


<210> 53
<211> 26
<212> DNA
<213> Artificial Sequence

<220>
<221> source
<222> 1..26
<223> /mol_type="unassigned DNA"
      /note="Oligonucleotide forward primer designed on
      the basis of NBS1 gene sequence enabling amplification of a region
      comprising a mutation change c.657del5 site"
      /organism="Artificial Sequence"

<400> 53

tttttacagt ttttacccac ctcttg                                          26

<210> 54
<211> 25
<212> DNA
<213> Artificial Sequence

<220>
<221> source
<222> 1..25
<223> /mol_type="unassigned DNA"
      /note="Oligonucleotide reverse primer designed
      on the basis of NBS1 gene sequence enabling amplification of
      a region comprising genetic change c.657del5 site"
      /organism="Artificial Sequence"

<400> 54
acagatttta agaaaccccg taatc                                           25

<210> 55
<211> 24
<212> DNA
<213> Artificial Sequence

<220>
<221> source
<222> 1..24
<223> /mol_type="unassigned DNA"
      /note="Oligonucleotide forward primer designed on
      the basis of BRCA1 gene sequence enabling amplification of a region
      comprising a mutation change c.185delAG site"
      /organism="Artificial Sequence"

<400> 55
cgttgtcatt agttctttgg tttg                                            24

<210> 56
<211> 22
<212> DNA
<213> Artificial Sequence

<220>
<221> source
<222> 1..22
<223> /mol_type="unassigned DNA"
      /note="Oligonucleotide reverse primer designed
      on the basis of BRCA1 gene sequence enabling amplification of
      a region comprising genetic change c.185delAG site"
      /organism="Artificial Sequence"

<400> 56
ggtcaattct gttcatttgc at                                              22

<210> 57

<211> 21
<212> DNA
<213> Artificial Sequence

<220>
<221> source
<222> 1..21
<223> /mol_type="unassigned DNA"
      /note="Oligonucleotide forward primer designed on
      the basis of BRCA1 gene sequence enabling amplification of a region
      comprising a mutation change p.C61G site"
      /organism="Artificial Sequence"

<400> 57
ggctcttaag ggcagttgtg a                                                21


<210> 58
<211> 21
<212> DNA
<213> Artificial Sequence

<220>
<221> source
<222> 1..21
<223> /mol_type="unassigned DNA"
      /note="Oligonucleotide reverse primer designed
      on the basis of BRCA1 gene sequence enabling amplification of
      a region comprising genetic change p.C61G site"
      /organism="Artificial Sequence"

<400> 58
tgtggttgct tccaacctag c                                                21


<210> 59
<211> 25
<212> DNA
<213> Artificial Sequence

<220>
<221> source
<222> 1..25
<223> /mol_type="unassigned DNA"
      /note="Oligonucleotide forward primer designed on
      the basis of BRCA1 gene sequence enabling amplification of a region
      comprising a mutation change c.4153delA site"
      /organism="Artificial Sequence"

<400> 59
ggaagcttct ctttctctta tcctg                                            25


<210> 60
<211> 21
<212> DNA
<213> Artificial Sequence

<220>
<221> source
<222> 1..21
<223> /mol_type="unassigned DNA"
      /note="Oligonucleotide reverse primer designed
      on the basis of BRCA1 gene sequence enabling amplification of
      a region comprising genetic change c.4153delA site"
      /organism="Artificial Sequence"

<400> 60
gcaaagggga gtggaataca g                                                21


<210> 61
<211> 25
<212> DNA
<213> Artificial Sequence

<220>
<221> source
<222> 1..25
<223> /mol_type="unassigned DNA"
      /note="Oligonucleotide forward primer designed on
      the basis of BRCA1 gene sequence enabling amplification of a region
      comprising a mutation change c.5382insC site"
      /organism="Artificial Sequence"

<400> 61
ggaagcttct ctttctctta tcctg                                            25


<210> 62
<211> 21
<212> DNA
<213> Artificial Sequence

<220>
<221> source
<222> 1..21
<223> /mol_type="unassigned DNA"
      /note="Oligonucleotide reverse primer designed
      on the basis of BRCA1 gene sequence enabling amplification of
      a region comprising genetic change c.5382insC site"
      /organism="Artificial Sequence"

<400> 62
gcaaagggga gtggaataca g                                                21


<210> 63
<211> 24
<212> DNA
<213> Artificial Sequence

<220>
<221> source
<222> 1..24
<223> /mol_type="unassigned DNA"

/note="Oligonucleotide forward primer designed on
the basis of CHEK2 gene sequence enabling amplification of a region
comprising a mutation change c.IVS2+1G>A site"
/organism="Artificial Sequence"

<400> 63
gccttcttag gctattttcc taca                                              24


<210> 64
<211> 22
<212> DNA
<213> Artificial Sequence

<220>
<221> source
<222> 1..22
<223> /mol_type="unassigned DNA"
/note="Oligonucleotide reverse primer designed
on the basis of CHEK2 gene sequence enabling amplification of
a region comprising genetic change c.IVS2+1G>A site"
/organism="Artificial Sequence"

<400> 64
ttattcaaag gacggcgttt tc                                                22


<210> 65
<211> 26
<212> DNA
<213> Artificial Sequence

<220>
<221> source
<222> 1..26
<223> /mol_type="unassigned DNA"
/note="Oligonucleotide forward primer designed on
the basis of CHEK2 gene sequence enabling amplification of a region
comprising a mutation change p.I157T site"
/organism="Artificial Sequence"

<400> 65
caatagaaac tgatctagcc tacgtg                                            26


<210> 66
<211> 32
<212> DNA
<213> Artificial Sequence

<220>
<221> source
<222> 1..32
<223> /mol_type="unassigned DNA"
/note="Oligonucleotide reverse primer designed
on the basis of CHEK2 gene sequence enabling amplification of
a region comprising genetic change p.I157T site"
/organism="Artificial Sequence"

<400> 66
aatattaagc cttagtgggt atccaccttt ct                                    32


<210> 67
<211> 20
<212> DNA
<213> Artificial Sequence

<220>
<221> source
<222> 1..20
<223> /mol_type="unassigned DNA"
      /note="Oligonucleotide forward primer designed on
      the basis of CHEK2 gene sequence enabling amplification of a region
      comprising a mutation change p.S428F site"
      /organism="Artificial Sequence"

<400> 67
cattgtgccc agcctagctc                                                  20


<210> 68
<211> 21
<212> DNA
<213> Artificial Sequence

<220>
<221> source
<222> 1..21
<223> /mol_type="unassigned DNA"
      /note="Oligonucleotide reverse primer designed
      on the basis of CHEK2 gene sequence enabling amplification of
      a region comprising genetic change p.S428F site"
      /organism="Artificial Sequence"

<400> 68
acttctgccc agacttcagg a                                                21